# EUROPEAN PATENT SPECIFICATION

(11) **EP 2 465 131 B1**
(45) Date of publication and mention of the grant of the patent: **07.06.2017**
(21) Application number: 10742905.2
(22) Date of filing: 29.07.2010
(51) Int. Cl.: H01J 35/14, H01J 35/26, A61B 6/00

(54) **X-RAY TUBE WITH INDEPENDENT X- AND Z- DYNAMIC FOCAL SPOT DEFLECTION**
RÖNTGENRÖHRE MIT UNABHÄNGIGER X- UND Z-DYNAMISCHER BRENNFLECKABLENKUNG
TUBE A RAYON X A DEVIATION X ET Z DYNAMIQUE DE FOYER ELECTRONIQUE INDEPENDANTE

(30) Priority: 13.08.2009 US 233505 P
(43) Date of publication of application: 20.06.2012
(73) Proprietor: Koninklijke Philips N.V., 5656 AE Eindhoven (NL)
(72) Inventor: KAUTZ, Allan, NL-5656 AE Eindhoven (NL); PERNO, Salvatore, NL-5656 AE Eindhoven (NL); BROOKS, Gary, NL-5656 AE Eindhoven (NL); AWAD, George, NL-5656 AE Eindhoven (NL); BUAN, Jose Angelo, NL-5656 AE Eindhoven (NL)
(74) Representative: van Velzen, Maaike Mathilde
(86) International application number: PCT/IB2010/053444
(87) International publication number: WO 2011/018729

(56) References cited:
- WO-A2-2008/044194
- DE-A1- 19 953 613
- JP-A- 61 153 934
- JP-A- 2009 158 138
- US-A- 4 718 076
- US-A- 4 748 650
- US-A1- 2005 029 957

## Description

### FIELD OF THE INVENTION

The present invention relates to an X-ray tube and an X-ray system including such a tube. Especially, the invention relates to an X-ray tube including means for deflection of the focal spot in x-direction and in y-direction (hence in z-direction). Further, the invention relates to a computer program for controlling an X-ray system including such an X-ray tube.

### BACKGROUND OF THE INVENTION

The concept of deflecting the focal spot to improve resolution of CT systems is well established. Initial applications using single line detectors employed a technique known as x-flying focal spot wherein the focal spot was oscillated in the lateral, or x-direction, about its rest position by approximately one-half pixel. This intentional introduction of parallax effectively doubles the resolution.

Double-x sampling is accomplished by oscillating the focal spot about its static position in a path tangential to the target focal track. The spot is displaced by approximately +/- 1/2 pixel either by using electrostatic grids or by an electromagnet. With double-x sampling, the electron beam/focal spot is deflected laterally to the filament/cathode at a prescribed frequency in the x-direction, tangential to the target focal track. Samples are taken at each extreme position so that one image contains information obtained at two viewing angles and contains virtually twice the information in a static image.

In recent years, the concept of double-z sampling, z-flying focal spot sampling or z-dynamic focal spot sampling has been established in the CT art as a mean of increasing resolution and decreasing artefacts. The method involves causing the focal spot to alternate between two z-positions, thus acquiring two slices per detector row, effectively double sampling in the z-direction. For example, with 32 rows of detectors, this technique effectively measures 64 slices.

Double-z sampling is accomplished essentially by moving the focal spot along a vector primarily directed in the y-direction on the target focal track. Because the focal track is at an angle with regard to the plane of the target (typically 7 or 8 degrees), movement of the focal spot in the y-direction causes it to also move in the z-direction, the distance being proportional to the sine of the target angle. The net effect of double-z sampling is to alternate the focal spot between two z-positions on the detector rows. This effectively acquires two overlapping slices per detector row, for example obtaining 64 slices in 32 rows. It is thus seen that double-z sampling can, in effect, double the slice capability of a CT scanner, turning a 32 slice scanner into a 64 slice scanner.

JP 2009 158138 A discloses an X-ray tube and an X-ray CT device capable of controlling an irradiation position of the X-ray with superior precision. In the X-ray tube, a pair of electrodes in which a coil filament to discharge an electron by energization and an anode to emit the X-ray by incidence of the electron are installed. This document also discloes that a coil is placed outside the X-ray tube housing.

### SUMMARY OF THE INVENTION

It is an object of the invention to include capability for both x-flying focal spot and z-flying focal spot with the same CT platform. It is a further object to include the capability of employing these techniques either together or independently.

This is achieved by the subject matter of each independent claim. Further embodiments are described in the respective dependent claims.

The object is generally solved by an X-ray tube adapted to generate X-ray beams, comprising a first deflection device for focal spot deflection in the x-direction, a second deflection device for focal spot deflection in the y-direction, and a cathode housing, wherein the first deflection device is mounted in the cathode housing and the second deflection device is mounted outside the housing, and wherein the first and second deflection devices are arranged separately from each other along the z-axis of the X-ray tube. The first deflection device is formed by electrostatic grids, and is mounted on the cathode cup. Even if two electrostatic grids may be usually utilized, it may be advantageous to have more than two grids.

The second deflection device includes an electromagnetic coil forming a dipole, and is mounted between a cathode and an anode of the X-ray tube. By mounting one deflection device on the cathode cup itself, internal to the X-ray tube, and by mounting the other deflection device around the electron beam path, external to the X-ray tube, interaction effects between the two devices are eliminated.

It is noted, that the simple dipole electromagnet requires only a small amount of magnetic material to produce required magnetic fields and hence the reluctance can be quite low, facilitating rapidly oscillating the magnetic field.

According to another embodiment of the invention, the second deflection device may also provide for focal spot deflection in x-direction, so that the second deflection device deflects the focal spot diagonally between the x- and y-direction. In this embodiment, the second deflection device is rotated slightly from true y-direction to produce a diagonal deflection of the focal spot. The first deflection device then may be used for (1) x-deflection when this alone is desired, (2) cancelling or modifying the x-portion of the diagonal deflection if y-deflection alone is desired or a different amount is desired in the diagonal deflection.

According to another embodiment, the first deflection device as well as the second deflection device may be operated to generate a static field or a dynamic field or a combination of the same, i.e. a static field superimposed by a dynamic field.

It is understood that both deflection devices may be operated with a variety of polarities. For example, the focal spot can be shaped and positioned by use of static fields and can be oscillated about this position by superimposing dynamic fields upon a static field.

Furthermore, the cathode of the X-ray tube may comprise more than one filament. For example, two filaments may be positioned in such a way that the corresponding electron beams impinge the anode at different focal spots. It may also be advantageous to have filaments with different sizes.

According to another aspect of the invention, an X-ray system comprises an X-ray tube as mentioned above, a detector for the detection of X-ray beams, and a processing unit. Such a system may further comprise a motor control unit controlling a motion of the X-ray tube and detector relative to an object of interest, and at the same time or independently controlling a motion of the object of interest itself. Such an X-ray system may be for example a computer tomography system.

The X-ray tube according to the invention provides for the following advantages:
▪ Electron emission may be cut off by applying sufficient negative potential to the first deflection device, i.e. for example to electrostatic grids.
▪ Focal spot shape changes are minimized from one position to another. Using two dipoles or quadrupole-dipole combinations leads to magnetic field interactions and distortions that produce different focal spot shapes in different deflected positions.
▪ Shorter anode-cathode spacing may be possible compared with two dipoles. Two dipoles will likely require more space in the direction of the tube axis. Shorter spacing allows for easier focusing.
▪ Higher power is possible. Since electrostatic deflection is faster than magnetic deflection, it avoids the case where the focal spot movement in the x-direction tracks a section of the focal track. When the focal spot moves along with the focal track, there is increased heating of that portion of the track. Therefore, power must be reduced to avoid overheating of the target focal track.
▪ Multiple focal spots may be possible using a single emitter without the use of a quadrupole. The first deflection device can be used to modify the focal spot width.

It will be understood, that the invention may also relate to a computer program for a processing unit of a system as mentioned above. The computer program may be adapted to control the deflection of the focal spot in x-direction as well as in y-direction (which results in a deflection in z-direction). The computer program may control the first deflection device as well as the separately arranged second deflection device.

On the other hand, the computer program may also include instruction for processing the data received from the detector as basis for images which may be illustrated on a monitor of the system, controlled by the computer program.

Furthermore, the computer program may also include instruction for controlling motion of the X-ray tube and detector relative to an object of interest, and for controlling motion of the object of interest itself relative to the X-ray tube and detector.

The computer program is preferably loaded into a working memory of a data processor. The data processor is thus equipped to carry out a method of the invention. Further, the invention relates to a computer-readable medium, such as a CD-ROM, at which the computer program may be stored. However, the computer program may also be presented over a network like the WorldWideWeb and can be downloaded into the working memory of the data processor from such a network.

It has to be noted that embodiments of the invention are described with reference to different subject-matter. In particular, some embodiments are described with reference to method type claims whereas other embodiments are described with reference to apparatus type claims. However, a person skilled in the art will gather from the above and the following description that, unless other notified, in addition to any combination of features belonging to one type of subject-matter also any combination between features relating to different subject-matters is considered to be disclosed with this application.

The aspects defined above and further aspects, features and advantages of the present invention can also be derived from the examples of embodiments to be described hereinafter and are explained with reference to examples of embodiments. The invention will be described in more detail hereinafter with reference to examples of embodiments but to which the invention is not limited.

### BRIEF DESCRIPTION OF THE DRAWINGS

Fig. 1 shows an overview of a system according to the invention.
Fig. 2 is a schematic illustration of an X-ray tube according to the invention.
Fig. 3 is an isometric view of an anode including a coordinate system as used in the context of this application.
Fig. 4 illustrates the relative positions of the electrostatic grids, the electromagnetic coils as well as the anode of an X-ray tube according to the invention.
Fig. 5 shows a flow chart with steps of a method for operating an X-ray system according to the invention.

The illustration in the drawings is schematically only and not to scale. It is noted in different figures, similar elements are provided with the same reference signs.

### DETAILED DESCRIPTION OF THE DRAWINGS

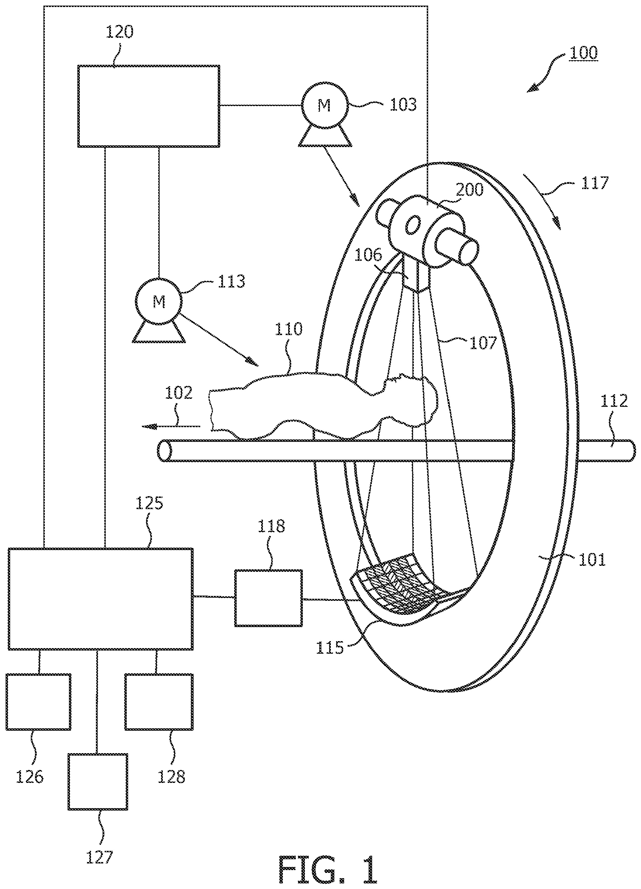

Fig. 1 shows an X-ray system 100 according to the invention. The X-ray system may be a computer tomography system, which is also called a CT scanner and in which the X-ray tube according to the invention can be used. The CT scanner 100 comprises a gantry 101, which is rotatable around a rotational axis 102. The gantry 101 is driven by means of a motor 103.

Reference number 200 designates a source of radiation such as an X-ray tube according to the invention, which may emit a polychromatic radiation 107. The CT scanner 100 further comprises an aperture system 106, which forms the X-radiation being emitted from the X-ray source 200 into a radiation beam 107. The spectral distribution of the radiation beam emitted from the radial source 200 may further be changed by a filter element (not shown), which may be arranged close to the aperture system 106.

The radiation beam 107, which may be a cone-shaped or a fan-shaped beam 107, is directed such that it penetrates a region of interest such as a head of a patient 110.

The patient 110 is positioned on a table 112. The patient's head is arranged in a central region of the gantry 101, which central region represents the examination region of the CT scanner 100. After penetrating the region of interest the radiation beam 107 impinges onto a radiation detector 115. In order to be able to suppress X-radiation being scattered by the patient's head and impinging onto the X-ray detector under an oblique angle there is provided and not depicted an anti-scatter-grid. The anti-scatter-grid is preferably positioned directly in front of the detector 115.

The X-ray detector 115 is arranged on the gantry 101 opposite to the X-ray tube 200. The detector 115 comprises a plurality of detector elements wherein each detector element is capable of detecting X-ray photons, which have passed through the head of the patient 110.

To improve the scanning process, the initial electron beam coming from the cathode in the X-ray tube 200, may be deflected by means of a first deflection device in an x-direction, and maybe deflected by means of a second deflection device in an y-direction which results in a deflection in z-direction. These elements will be described in more detail below.

During scanning the region of interest, the X-ray source 200, the aperture system 106 and the detector 115 are rotated together with the gantry 101 in a rotation direction indicated by an arrow 117. For rotation of the gantry 101, the motor 103 is connected to a motor control unit 120, which itself is connected to a data processing device 125. The data processing device 125 includes a reconstruction unit, which maybe realized by means of hardware and/or by means of software constituting a part of a computer program according to the invention. The reconstruction unit is adapted to reconstruct a 3D image based on a plurality of 2D images obtained under various observation angles, which may be additionally adjusted by means of the electron beam deflection in the X-ray tube 200.

Furthermore, the data processing device 125 serves also as a control unit, which communicates with the motor control unit 120 in order to coordinate the movement of the gantry 101 with the movement of the table 112. A linear displacement of the table 112 is carried out by a motor 113, which is also connected to the motor control unit 120.

During operation of the CT scanner 100 the gantry 101 may rotate and in the same time the table 112 may shift linearly parallel to the rotational axis 102 such that a helical scan of the region of interest may be performed. It should be noted that it may be also possible to perform a circular scan, where there is no displacement in a direction parallel to the rotational axis 102, but only the rotation of the gantry 101 around the rotational axis 102. Thereby, slices of the head may be measured with high accuracy. A larger three-dimensional representation of the patient's head may be obtained by sequentially moving the table 112 in discrete steps parallel to the rotational axis 102 after at least one-half gantry rotation has been performed for each discrete table position.

The detector 115 may be coupled to a pre-amplifier 118, which itself may be coupled to the data processing device 125. The processing device 125 is capable, based on a plurality of different X-ray projection data sets, which have been acquired at different projection angles, to reconstruct a 3D representation of the patient's head.

In order to observe the reconstructed 3D representation of the patient's head a display 126 is provided, which is coupled to the data processing device 125. Additionally, arbitrary slices of a perspective view of the 3D representation may also be printed out by a printer 127, which is also coupled to the data processing device 125. Further, the data processing device 125 may also be coupled to a picture archiving and communication system 128.

It should be noted that the monitor 126, the printer 127 and/or the other devices supplied in the CT scanner 100 might be arranged local to the computer tomography apparatus 100. Alternatively, these components maybe remote from the CT scanner 100, such as elsewhere within an institution or hospital, or in an entirely different location linked to the CT scanner 100 by use of one or more configurable networks, such as the Internet, virtual private networks and so forth.

Finally, the processing device 125 is also connected with the X-ray tube 200. By way of this connection, the processing device 125 is capable of controlling the focal spot deflection in x- and y-direction by means of the first and second deflection devices, respectively.

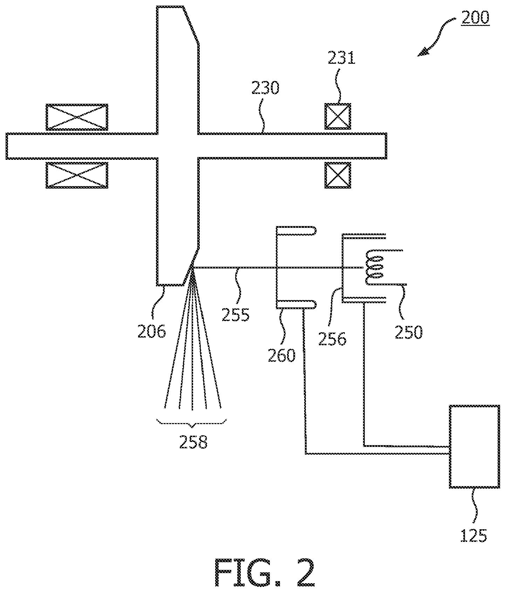

Fig. 2 shows an X-ray tube 200, which is adapted to generate X-rays originated from different X-ray focal spots. The X-ray tube 200 comprises an anode 206 having a shaft 230. The shaft 230 is guided in such a manner that the shaft 230 may be rotated around the z-axis. A rotational drive 231 is provided in order to allow for a rotational movement of the anode 206. The drive 231 may interact with the shaft 230 by means of a mechanical and/or a magnetic interaction point.

The X-ray tube 200 further comprises an electron source 250, which is arranged laterally with respect to the z-axis. According to the embodiment described here, the electron source may be a hot cathode 250, which during operation generates an electron beam 255. The electron beam impinges onto a top surface of the anode 206. Thereby, a focal spot is defined. The top surface is orientated oblique with respect to the z-axis such that from the focal spot an X-ray beam 258 projects radially outwards from the z-axis.

In order to control the exact position of the focal spot the X-ray tube 200 further comprises a first electron deflection device 256, which is adapted to deflect the electron beam 255 in the x-direction. The first electron deflection device 256 may be formed for example by electrostatic grids. The first electron deflection device 256 is coupled to the control unit 125, which provides the necessary electric signals to the first electron deflection device 256. For example, one of the grids may be energized with -100V and the other one of the grids maybe energized with -50V.

Further, the X-ray tube 200 comprises a second electron deflection device 260, which may be formed by two electromagnetic coils. Such coils are shown mounted around the neck of the cathode, between the cathode cup and the anode. The coils constitute a dipole which is positioned so that its magnetic force produces deflection in the y-, and hence z-direction. The second electron deflection device is also coupled to the control unit 125. Therefore, Fig. 2 illustrates a combination of two separate deflection devices to produce independent x and y (z) focal spot deflection.

It is noted that the dipole may also be constituted by a U-shaped magnetic element with only one electromagnetic coil located around the bottom of the 'U' between the legs of the 'U'.

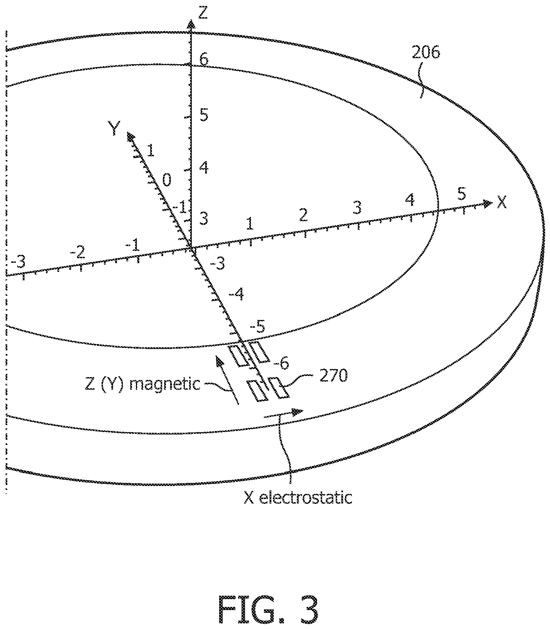

Fig. 3 illustrates first of all the CT coordinate system, showing the conventional directions of x, y and z. It is noted, that this CT coordinate system is referenced to a patient, wherein the x-direction is lateral to the patient, the y-direction is vertical to the patient, and the z-direction is along the length of the patient.

Also shown in Fig. 3 are four deflected focal spot positions 270 on the target area of the anode 206. The deflection in the z-direction is produced by deflecting the electron beam in the y-direction. This causes the beam to walk up the angled track, causing the focal spot to move in the z-direction.

The x-deflection results in an introduced parallax to improve resolution, and the z-deflection provides a double-slice capability.

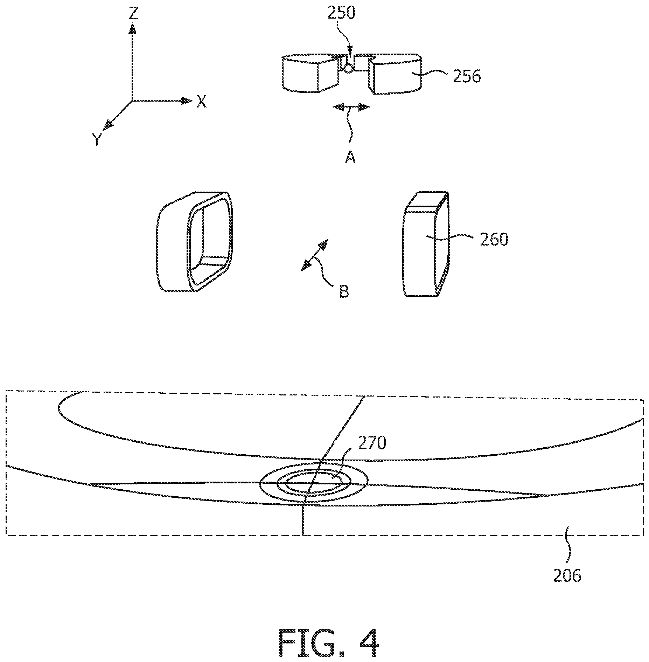

Fig. 4 shows a detailed view of the arrangement of the first deflection device, the second deflection device and a section of the anode. Furthermore, a reference coordinate system is illustrated. The electron emitting cathode 250 is shown with the first deflection device, here two electrostatic grids 256, mounted on either side of a cathode so that the force due to the electrostatic field produces deflection in the x-direction (indicated by arrow A). Coming from the cathode, the electron will pass subsequently the magnetic field of the second deflection device, here electromagnetic coils 260, which is positioned to induce a deflection of the electron in y-direction (indicated by arrow B). Finally, each electron will impinge on the target area 270 of the anode.

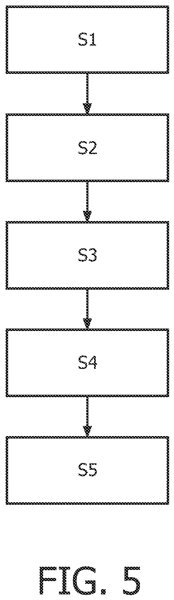

Fig. 5 shows a flow chart with steps of a method for operating an X-ray system as described above. These steps are implemented as instructions in a computer program according to the invention. It will be understood, that the steps described with respect to the method, are major steps, wherein these major steps might be differentiated or divided into several sub steps. Furthermore, there might be also sub steps between these major steps. Therefore, a step is only mentioned, if said step is important for the understanding of the principles of the method according to the invention.

In step S1, the X-ray tube and the detector are positioned relative to the object of interest, by means of controlling the motor 103 and 113.

In step S2, the electron beam emitted by the cathode 250 in the X-ray tube 200, is deflected in x-direction, by means of controlling the first deflection device 256.

In step S3, the electron beam coming from the first deflection device 256, is deflected in y-direction, by means of controlling the second deflection device 260.

In step S4, the electron beam leaving the X-ray tube and penetrating the object of interest, impinges the detector elements of the detector.

Finally, in step S5, images are generated by the processing unit 125 on the basis of the signals received from the detector elements.

While the invention has been illustrated and described in detail in the drawings and foregoing description, such illustration and description are to be considered illustrative or exemplary and not restrictive. The invention is not limited to the disclosed embodiments.

Other variations to the disclosed embodiments can be understood and effected by those skilled in the art in practising the claimed invention, from a study of the drawings, the disclosure and the appended claims. In the claims the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE SIGNS:

- 100: X-ray system
- 101: gantry
- 102: rotational axis
- 103, 113: motor
- 106: aperture system
- 107: radiation
- 110: patient
- 112: table
- 115: detector
- 117: rotation direction
- 118: pre-amplifier
- 120: motor control unit
- 125: processing device
- 126: display
- 127: printer
- 128: archiving /communication system
- 200: X-ray tube
- 206: anode
- 230: shaft
- 231: rotational drive
- 250: electron source /cathode
- 255: electron beam
- 256: first electron deflection device
- 258: X-ray beam
- 260: second electron deflection device
- 270: focal spot position /target area

## Claims

1. An X-ray tube (200) adapted to generate X-ray beams, comprising
a first deflection device (256) for focal spot deflection in x-direction,
a second deflection device (260) for focal spot deflection in y-direction, and
a cathode housing,
wherein the first deflection device is mounted in the cathode housing and the second deflection device is mounted outside the housing, and wherein the first and second deflection devices are arranged separately from each other along a z-axis of the X-ray tube and
wherein the first deflection device includes electrostatic grids (256), and is mounted on a cathode cup of the X-ray tube and
wherein the second deflection device includes an electromagnetic coil (260) forming a dipole, and is mounted between a cathode (250) and an anode (206) of the X-ray tube.

2. The X-ray tube of claim 1, wherein the second deflection device (260) also provide for focal spot deflection in x-direction, so that the second deflection device deflect the focal spot diagonally.

3. The X-ray tube of claim 1, wherein the first deflection device (256) is operated to generate a field out of the group consisting of a static field, a dynamic field and a static field superimposed by a dynamic field.

4. The X-ray tube of claim 1, wherein the second deflection device (260) is operated to generate a field out of the group consisting of a static field, a dynamic field and a static field superimposed by a dynamic field.

5. The X-ray tube of claim 1, comprising a cathode (250) having one or more filaments.

6. An X-ray system (100) comprising
an X-ray tube (200) according to claim 1,
a detector (115) for the detection of X-ray beams, and
a processing unit (125).

7. The X-ray system of claim 8, further comprising a motor control unit (120) controlling a motion of the X-ray tube (200) and detector (115) relative to an object of interest (110), and controlling a motion of the object of interest.

8. A computer program including instructions which, when executed on a processing unit (125) of a system (100) according to claim 8, causes the first deflection device (256) of the X-ray tube (200) of the X-ray system (100) to deflect the focal spot of the electron beam in the x-direction, and causes the second deflection device (260) of the X-ray tube of the X-ray system to deflect the focal spot of the electron beam in the y-direction.

9. The computer program of claim 10, further including instructions for processing the data received from the detector (115), to generate images of an object of interest, and for illustrating the images on a display (126) of the system.

10. The computer program of claim 10, further including instructions for controlling motion of the X-ray tube (200) and detector (115) relative to an object of interest (110), and for controlling motion of the object of interest relative to the X-ray tube and detector.

## Patentansprüche

1. Röntgenröhre (200), die dafür vorgesehen ist, Röntgenstrahlen zu erzeugen, umfassend:
eine erste Ablenkungsvorrichtung (256) zur Brennfleckablenkung in x-Richtung,
eine zweite Ablenkungsvorrichtung (260) zur Brennfleckablenkung in y-Richtung, und
ein Kathodengehäuse,
wobei die erste Ablenkungsvorrichtung in dem Kathodengehäuse montiert ist und die zweite Ablenkungsvorrichtung außerhalb des Gehäuses montiert ist, und wobei die erste Ablenkungsvorrichtung und die zweite Ablenkungsvorrichtung getrennt voneinander auf einer z-Achse der Röntgenröhre angeordnet sind, und
wobei die erste Ablenkungsvorrichtung elektrostatische Gitter (256) umfasst und an einem Kathodenbecher der Röntgenröhre montiert ist, und
wobei die zweite Ablenkungsvorrichtung eine elektromagnetische Spule (260) umfasst, die einen Dipol bildet, und zwischen einer Kathode (250) und einer Anode (206) der Röntgenröhre montiert ist.

2. Röntgenröhre nach Anspruch 1, wobei die zweite Ablenkungsvorrichtung (260) auch eine Brennfleckablenkung in x-Richtung bereitstellt, so dass die zweite Ablenkungsvorrichtung den Brennfleck diagonal ablenkt.

3. Röntgenröhre nach Anspruch 1, wobei die erste Ablenkungsvorrichtung (256) betätigt wird, um ein Feld aus der Gruppe bestehend aus einem statischen Feld, einem dynamischen Feld und einem statischen Feld überlagert durch ein dynamisches Feld zu erzeugen.

4. Röntgenröhre nach Anspruch 1, wobei die zweite Ablenkungsvorrichtung (260) betätigt wird, um ein Feld aus der Gruppe bestehend aus einem statischen Feld, einem dynamischen Feld und einem statischen Feld überlagert durch ein dynamisches Feld zu erzeugen.

5. Röntgenröhre nach Anspruch 1, umfassend eine Kathode (250) mit einem oder mehreren Heizdrähten.

6. Röntgensystem (100), umfassend:
eine Röntgenröhre (200) nach Anspruch 1,
einen Detektor (115) zur Detektion von Röntgenstrahlen, und
eine Verarbeitungseinheit (125).

7. Röntgensystem nach Anspruch 6, weiterhin umfassend eine Motorsteuereinheit (120), die eine Bewegung der Röntgenröhre (200) und des Detektors (115) in Bezug auf ein interessierendes Objekt (110) steuert und eine Bewegung des interessierenden Objekts steuert.

8. Computerprogramm umfassend Anweisungen, die, wenn sie auf einer Verarbeitungseinheit (125) eines Systems (100) nach Anspruch 6 ausgeführt werden, die erste Ablenkungsvorrichtung (256) der Röntgenröhre (200) des Röntgensystems (100) veranlassen, den Brennfleck des Elektronenstrahls in der x-Richtung abzulenken, und die zweite Ablenkungsvorrichtung (260) der Röntgenröhre des Röntgensystems veranlassen, den Brennfleck des Elektronenstrahls in der y-Richtung abzulenken.

9. Computerprogramm nach Anspruch 8, weiterhin umfassend Anweisungen zum Verarbeiten der von dem Detektor (115) empfangenen Daten, um Bilder eines interessierenden Objekts zu erzeugen und um die Bilder auf einer Anzeige (126) des Systems darzustellen.

10. Computerprogramm nach Anspruch 8, weiterhin umfassend Anweisungen zum Steuern der Bewegung der Röntgenröhre (200) und des Detektors (115) in Bezug auf ein interessierendes Objekt (110) und zum Steuern der Bewegung des interessierenden Objekts in Bezug auf die Röntgenröhre und den Detektor.

## Revendications

1. Tube à rayons X (200) apte à générer des faisceaux de rayons X, comprenant :
un premier dispositif de déviation (256) pour une déviation de foyer dans un sens x,
un deuxième dispositif de déviation (260) pour une déviation de foyer dans un sens y, et
un logement de cathode,
dans lequel le premier dispositif de déviation est monté dans le logement de cathode et le deuxième dispositif de déviation est monté à l'extérieur du logement, et dans lequel les premier et deuxième dispositifs de déviation sont agencés séparément l'un de l'autre le long d'un axe z du tube à rayons X, et
dans lequel le premier dispositif de déviation comprend des grilles électrostatiques (256) et est monté sur une coupe de cathode du tube à rayons X, et
dans lequel le deuxième dispositif de déviation comprend une bobine électromagnétique (260) formant un dipôle, et est monté entre une cathode (250) et une anode (206) du tube à rayons X.

2. Tube à rayons X selon la revendication 1, dans lequel le deuxième dispositif de déviation (260) fournit également une déviation de foyer dans le sens x, de sorte que le deuxième dispositif de déviation dévie le foyer diagonalement.

3. Tube à rayons X selon la revendication 1, dans lequel le premier dispositif de déviation (256) est actionné pour générer un champ dans le groupe se composant d'un champ statique, un champ dynamique et un champ statique sur lequel est superposé un champ dynamique.

4. Tube à rayons X selon la revendication 1, dans lequel le deuxième dispositif de déviation (260) est actionné pour générer un champ dans le groupe se composant d'un champ statique, un champ dynamique et un champ statique sur lequel est superposé un champ dynamique.

5. Tube à rayons X selon la revendication 1, comprenant une cathode (250) comportant un ou plusieurs filaments.

6. Système à rayons X (100) comprenant :
un tube à rayons X (200) selon la revendication 1,
un détecteur (115) pour la détection de faisceaux de rayons X, et
une unité de traitement (125).

7. Système à rayons X selon la revendication 6, comprenant en outre une unité de commande de moteur (120) commandant un mouvement du tube à rayons X (200) et du détecteur (115) par rapport à un objet d'intérêt (110), et commandant un mouvement de l'objet d'intérêt.

8. Programme informatique comprenant des instructions qui, lorsqu'elles sont exécutées sur une unité de traitement (125) d'un système (100) selon la revendication 6, amènent le premier dispositif de déviation (256) du tube à rayons X (200) du système à rayons X (100) à dévier le foyer du faisceau d'électrons dans le sens x, et amènent le deuxième dispositif de déviation (260) du tube à rayons X du système à rayons X à dévier le foyer du faisceau d'électrons dans le sens y.

9. Programme informatique selon la revendication 8, comprenant en outre des instructions pour traiter les données reçues en provenance du détecteur (115), pour générer des images d'un objet d'intérêt, et pour illustrer les images sur un affichage (126) du système.

10. Programme informatique selon la revendication 8, comprenant en outre des instructions pour commander le mouvement du tube à rayons X (200) et du détecteur (115) par rapport à un objet d'intérêt (110), et pour commander le mouvement de l'objet d'intérêt par rapport au tube à rayons X et au détecteur.
